**Europäisches Patentamt**

**European Patent Office**

**Office européen des brevets**

(11) Veröffentlichungsnummer : **0 071 169**
**B1**

(12) **EUROPÄISCHE PATENTSCHRIFT**

(45) Veröffentlichungstag der Patentschrift :
30.12.86

(21) Anmeldenummer : 82106567.9

(22) Anmeldetag : 21.07.82

(51) Int. Cl.⁴ : **C 08 J 9/28, B 01 D 13/04,**
**D 01 D 5/24, G 01 N 33/50,**
**G 01 N 33/53, A 61 L 15/03,**
**B 41 M 5/26, C 12 Q 1/00**

(54) **Offenporig-mikroporös ausgebildeter Formkörper mit inhärenter latenter Strukturumwandelbarkeit.**

(30) Priorität : 28.07.81 DE 3129745

(43) Veröffentlichungstag der Anmeldung :
09.02.83 Patentblatt 83/06

(45) Bekanntmachung des Hinweises auf die Patenterteilung : 30.12.86 Patentblatt 86/52

(84) Benannte Vertragsstaaten :
BE CH DE FR GB IT LI NL SE

(56) Entgegenhaltungen :
EP-A- 0 012 557
DE-A- 2 632 185
US-A- 2 468 664
US-A- 3 445 434
US-A- 3 615 024
US-A- 4 049 589

(73) Patentinhaber : HOECHST AKTIENGESELLSCHAFT
Postfach 80 03 20
D-6230 Frankfurt am Main 80 (DE)

(72) Erfinder : Walch, Axel, Dr.
Hans-Sachs-Strasse 5
D-6000 Frankfurt/M. (DE)
Erfinder : Seifried, Walter, Dr.
Am Güldenplan 9
D-6200 Wiesbaden 1 (DE)
Erfinder : Michel, Wolfgang
Am Hohen Stein 24
D-6200 Wiesbaden 1 (DE)
Erfinder : Kuhls, Jürgen, Dr.
Unghausen 16a
D-8263 Burghausen (DE)
Erfinder : Wildhardt, Jürgen
Am Birnbusch 14
D-6274 Hünstetten (DE)

## Beschreibung

Die Erfindung betrifft offenporig-mikroporös ausgebildete Formkörper mit inhärenter latenter Strukturumwandelbarkeit, Verfahren zu ihrer Herstellung sowie Verfahren zur Umwandlung ihrer Struktur.

Die Erfindung umfaßt auch besondere Verwendungen der Formkörper.

Die Bezeichnung « Formkörper » soll im Rahmen der vorliegenden Erfindungsbeschreibung und der Ansprüche Folien sowie rohrförmige Gebilde, wie Schläuche sowie Hohlfäden (Kapillaren), umfassen. Definitionsgemäß umfaßt die Bezeichnung « Formkörper » auch Beschichtungen.

Unter mikroporösen Formkörpern sind im Rahmen der vorliegenden Erfindungsbeschreibung und der Ansprüche Formkörper zu verstehen, die effektive Poren der angegebenen Größe besitzen. Die erfindungsgemäßen offenporigmikroporösen Formkörper werden nachfolgend kurz als « offenporige Formkörper » bezeichnet.

Erfindungsgemäße Formkörper werden auch als Zwischenerzeugnis bezeichnet.

Es sind offenporig-mikroporöse Kunststoffolien bekannt, beispielsweise solche aus Polyamid, Polysulfon oder Polyvinylidenfluorid (US-PS-3 61-5 024), die nach dem sogenannten Phaseninversionsverfahren durch Gießen einer Kunststofflösung zu einem flüssigen Film und anschließendem Koagulieren des im flüssigen Film gelösten Kunststoffes unter Ausbildung eines formbeständigen mikroporösen Kunststoffilmes hergestellt sind. Die bekannten Folien sind befähigt, in ihren Poren Flüssigkeit aufzunehmen.

Die bekannten mikroporösen Folien sind durch Strukturumwandlung jedoch nur unbefriedigend in physikalisch bzw. optisch praktisch homogenen und transparenten Zustand überführbar, weil diese nicht spontan, d. h. innerhalb eines sehr kurzen Zeitraumes durchführbar ist, bzw. der Temperaturbereich, in dem die Strukturumwandlung stattfindet, unvorteilhaft breit ist.

Unter dem eingetragenen Warenzeichen Poroplastic® ist eine Folie auf Basis von Cellulosetriacetat im Handel, die Flüssigkeit aufzunehmen vermag.

Infolge ihrer extrem geringen Porengröße ist die im Handel befindliche Folie transparent. Die Porenstruktur der Folie bricht infolge Schrumpfung der Folie irreversibel zusammen, wenn die Flüssigkeit enthaltende Folie austrocknet ; sie läßt sich dann nicht mehr rehydratisieren und absorbiert auch keine Flüssigkeit mehr.

Der Erfindung liegt die Aufgabe zugrunde, offenporig aufgebaute, strukturbedingt weiße, Formkörper vorzuschlagen, deren Struktur erhalten bleibt, wenn deren Poren mit Flüssigkeit beladen und sie anschliessend getrocknet werden und die durch gezielt abstufbare Umwandlung ihrer Struktur, im Grenzfall bis zur Transparenz, lichtdurchlässig gemacht werden können, wobei nach Maßgabe zunehmender Lichtdurchlässigkeit im Formkörper infolge Umwandlung seiner Struktur dessen freie Porosität verringert wird, wobei die Strukturumwandlung im Grenzfall bis zur praktisch physikalischen bzw. optischen Homogenität der Formkörper steigerbar ist und die Strukturumwandlung derselben durch geeignete Maßnahmen innerhalb eines engen Umwandlungsbereiches und in kurzer Zeit durchgeführt werden kann.

Die der Erfindung zugrundeliegende Aufgabe wird durch die in Anspruch 1 und 2 angegebenen Formkörper gelöst. Besonders vorteilhafte Ausgestaltungen der gegenständlichen Erfindung sind in den auf Anspruch 1 rückbezogenen Unteransprüchen konkretisiert. Die Ansprüche 5 und 6 geben Verfahren zur Herstellung erfindungsgemäßer Formkörper an. Die Ansprüche 10 bis 11 betreffen Verfahren zur Umwandlung der Struktur der gegenständlichen Erfindung nach einem der Ansprüche 1 bis 4. Die Ansprüche 7 bis 9 beziehen sich auf Verwendungen der gegenständlichen Erfindungen nach Anspruch 1 bis 4.

Erfindungsgemäße Formkörper sind als Ausgangsprodukt zur Herstellung von Formkörpern, insbesondere Folien, geeignet, die wenigstens innerhalb diskreter Bereiche praktisch physikalisch homogen sind bzw. erscheinen, wobei diese Bereiche gegebenenfalls Fluid enthalten. Der flüchtige Anteil des Fluids kann aus den physikalisch praktisch homogenen Bereichen der Formkörper nur durch Permeation austreten. Die Permeationsdauer hängt dabei von der chemischen Eigenart des flüchtigen Anteils des Fluids ab.

Die Erfindung betrifft ferner die Verwendung einer Folie nach einem der Ansprüche 1 und 2 als beschreibbares oder bedruckbares Flächengebilde, bei dem die auf dieses aufgebrachte Schrift oder Information, je nachdem, ob das dazu verwendete flüssige Schreibmaterial je nach Art seines chemischen Aufbaues die Struktur der Folie permanent umwandelt oder nicht, entweder dauerhaft erhalten bleibt oder innerhalb eines bestimmten Zeitraumes nach Aufbringen der Schrift nicht mehr sichtbar ist. Die Schrift oder die Information kommt dabei durch definiert erzeugte optische Homogenität des Formkörpers zustande. Die Erfindung kann dadurch auch zur Vermittlung indirekter Information genutzt werden (z. B. Kopiervorlagen, Pausvorlagen, lichtbedingte Wirkungen).

Die erfindungsgemäße Folie bzw. die Wandung des erfindungsgemäßen rohrförmigen Gebildes hat jeweils eine Dicke vorteilhaft im Bereich von 0,5 bis 800 μm.

Die Folie kann selbsttragend sein oder sich auf einem Trägerflächengebilde, beispielsweise einer streckorientierten Folie aus Polyester oder einer Folie aus weichmacherfreiem Polyvinylchlorid befinden.

Die Folie bzw. die Wandung des rohrförmigen Gebildes besitzt offenporige Struktur mit Poren von effektivem Durchmesser im Bereich von 0,002 bis 10 μm. Die Folie bzw. die Wandung des rohrförmigen Gebildes kann auch anisotrop-porös ausgebildet sein.

Die Formkörper sind infolge ihrer angegebenen Struktur bei Tageslicht weiß.

Chemisch-stofflich sind die Formkörper dadurch charakterisiert, daß sie zu wenigstens 70 Gew.-% aus filmbildendem, synthetischem Copolymerisat (1) bestehen, das zu 20 bis 80 Gew.-%, bezogen auf das Gesamtgewicht desselben, aus copolymerisiertem fluoriertem Olefin, bevorzugt copolymerisiertem fluoriertem Ethylen oder copolymerisiertem fluoriertem Propylen, insbesondere jedoch aus copolymerisiertem perfluoriertem Ethylen, zu 0 bis 40 Gew.-%, bezogen auf das Gesamtgewicht des Copolymerisats, aus copolymerisiertem Olefin, bevorzugt copolymerisiertem Ethylen oder copolymerisiertem Propylen und zu 80 bis 20 Gew.-%, bezogen auf das Gesamtgewicht des Copolymerisats, aus copolymerisiertem Vinylacetat aufgebaut ist, wobei wenigstens 5 Gew.-%, vorzugsweise mehr als 80 Gew.-%, der Acetatgruppen im angegebenen Copolymerisat, bezogen auf deren Gesamtmenge in diesem, nach erfolgter Copolymerisation der bezeichneten Comonomeren zum angegebenen Copolymerisat durch Verseifung derselben in OH-Gruppen umgewandelt sind. Definitionsgemäß werden unter Copolymerisat auch Blockcopolymerisate oder Mischungen derselben verstanden.

Der Formkörper kann bis zu 30 Gew.-%, bezogen auf sein Gesamtgewicht, Polymerisate enthalten, die sich von den ihn im wesentlichen aufbauenden bezeichneten Copolymerisaten bezüglich ihres qualitativen chemischen Aufbaus unterscheiden, wie bspw. Polyvinylidenfluorid, polares Polyolefin oder Silikone oder Mischungen der genannten Polymeren.

Bevorzugt enthalten die Formkörper Copolymerisat (2), das zu 30 bis 70 Gew.-%, bezogen auf sein Gesamtgewicht, aus copolymerisiertem Tetrafluorethylen, zu 0 bis 20 Gew.-%, bezogen auf das Gesamtgewicht des Copolymerisats, aus copolymerisiertem Ethylen und zu 70 bis 30 Gew.-%, bezogen auf das Gesamtgewicht des Copolymerisats aus copolymerisiertem Vinylacetat aufgebaut ist, wobei mehr als 5 Gew.-%, bevorzugt mehr als 80 Gew.-%, der Gesamtmenge der im bezeichneten Copolymerisat enthaltenen Acetatgruppen, durch Verseifung derselben nach erfolgter Copolymerisation der bezeichneten Comonomeren zum angegebenen Copolymerisat in OH-Gruppen umgewandelt sind.

Vorteilhaft enthalten die Formkörper z. B. Copolymerisat (3), das zu 45 Gew.-% aus copolymerisiertem Tetrafluorethylen, zu 7 Gew.-% aus copolymerisiertem Ethylen und zu 48 Gew.-% aus copolymerisiertem Vinylacetat aufgebaut ist, wobei mehr als 80 % der Acetatgruppen des Copolymerisats nach Herstellung desselben zu OH-Gruppen verseift sind.

Andere bevorzugte Formkörper enthalten Copolymerisat (4), das zu 58 Gew.-% aus copolymerisiertem Tetrafluorethylen, zu 7 Gew.-% aus copolymerisiertem Ethylen und zu 35 Gew.-% aus copolymerisiertem Vinylacetat aufgebaut ist, wobei mehr als 80 % der Acetatgruppen des Copolymerisats nach dessen Herstellung zu OH-Gruppen verseift sind.

Besondere Formkörper enthalten zweikomponentiges Copolymerisat (5), es ist zu 62 Gew.-%, bezogen auf das Gesamtgewicht des Copolymerisates, aus copolymerisiertem Tetrafluorethylen und zu 38 Gew.-%, aus copolymerisiertem Vinylacetat aufgebaut, wobei mehr als 80 % der Acetatgruppen des Copolymerisates nach dessen Herstellung durch Verseifung in OH-Gruppen übergeführt sind.

Die gewichtsprozentualen Angaben zu den in den beispielhaften Copolymerisaten jeweils enthaltenen Mengen bezeichneter copolymerisierter Comonomerer gelten jeweils mit der Maßgabe, daß deren Gesamtsumme jeweils 100 % beträgt.

Copolymerisate des genannten chemischen Aufbaues sind nach Verfahren herstellbar, die dem Fachmann geläufig sind (US-PS-3 445 434 sowie US-PS-2 468 664). Die vorstehend angegebenen Copolymerisate sind per se nicht Gegenstand vorliegender Erfindung.

Infolge des chemischen Aufbaues der Molekülketten des Copolymerisates, das die Formkörper bildet, sind diese sowohl oleophob als auch olephil. Sie sind dadurch (auch nach Umwandlung ihrer Struktur) verträglich mit unterschiedlichen sie kontaktierenden Stoffen oder Flüssigkeiten, die somit rasch miteinander ausgetauscht werden können.

Erfindungsgemäße Formkörper lassen sich nach Beladung ihrer Poren mit Flüssigkeit wieder trocknen und sind danach wieder verwendbar; ihr Eigenschaftsprofil wird durch den Trockenvorgang nicht wesentlich verändert. Die Struktur der Formkörper bleibt demnach aufgrund ihres Aufbaues stabil, wenn ihre Poren mit Flüssigkeit ausgefüllt werden und man die Flüssigkeit zu einem späteren Zeitpunkt wieder aus den Poren vertreibt oder sie aus diesen verdunsten läßt.

Erfindungsgemäße Formkörper sind durch inhärente latente, gezielt abstufbare Umwandelbarkeit ihrer Struktur charakterisiert.

Nach Umwandlung ihrer Struktur sind die Formkörper physikalisch bzw. optisch praktisch homogen und dementsprechend transparent.

Erfindungsgemäße Formkörper sind als Zwischenprodukte zur Herstellung physikalisch bzw. optisch praktisch homogener, transparenter Formkörper geeignet.

Die Bezeichnung, daß strukturumgewandelte Formkörper physikalisch praktisch homogen sind, bedeutet definitionsgemäß, daß die Folie bzw. die Wandung des rohrförmigen Gebildes praktisch keine lichtbrechenden, freien Poren besitzt. Die Bezeichnung, daß strukturumgewandelte Formkörper optisch homogen sind, bedeutet definitionsgemäß auch, daß ihre Poren oder Hohlräume weitgehend mit Fluid gefüllt sind, so daß die Folie transparent bzw. homogen erscheint.

Die Strukturumwandlung der Formkörper ist durch die Wahl der Bedingungen, unter denen man diese vornimmt, gezielt steuerbar. Die Strukturumwandlung kann über Zwischenstufen unterschiedlicher Porosität der Folie bzw. der Wandung des rohrförmigen Gebildes bzw. unterschiedlicher Lichtdurchlässigkeit derselben im Grenzfall bis zur physikalisch praktischen Homogenität bzw. Transparenz der Formkörper geführt werden.

Mit zunehmender Lichtdurchlässigkeit der Formkörper nimmt deren freie Porosität ab, d. h. die Anzahl bzw. Größe der lichtbrechenden Hohlräume, die sich in der Folie bzw. in der Wandung des rohrförmigen Gebildes befinden, nimmt ab.

Der erfindungsgemäße Formkörper wird wegen seiner Umwandelbarkeit in transparenten Zustand als « latent transparent » bezeichnet.

Die Struktur erfindungsgemäßer Formkörper läßt sich über Zwischenstufen dadurch bis zur Transparenz bzw. physikalisch praktischen Homogenität umwandeln, daß man sie einer geeigneten physikalischen oder chemischen Maßnahme oder einer Kombination beider unterwirft.

Nachfolgend sind Maßnahmen angegeben, die zur Strukturumwandlung erfindungsgemäßer Formkörper geeignet sind, z. B.

1. Einwirkung von Wärme auf erfindungsgemäße Formkörper. Je nach chemischem Aufbau des den Formkörper jeweils bildenden Copolymerisates, wird der Formkörper durch Wärmeeinwirkung auf eine Temperatur im Bereich zwischen 50 und 220 °C erhitzt. Dabei erfolgt die Umwandlung der Struktur der Formkörper im angegebenen Temperaturbereich jeweils innerhalb einer relativ engen Temperaturzone von weniger als 10 °C und läuft spontan ab.

2. Beaufschlagung erfindungsgemäßer Formkörper mit gasförmigem oder flüssigem Medium, das befähigt ist, das Copolymerisat, aus dem der Formkörper besteht, anzulösen, beispielsweise mit Aceton in flüssiger oder Dampfform. Die Dauer der Strukturumwandlungsmaßnahme hängt von der Konzentration des flüssigen Mediums und von seiner Temperatur ab ; die Strukturumwandlung erfolgt praktisch spontan.

Definitionsgemäß soll unter praktisch spontanem Ablauf der Strukturumwandlung verstanden werden, daß diese sich innerhalb eines Zeitraumes von Sekunden vollzieht.

Die Strukturumwandlung erfindungsgemäßer Formkörper, insbesondere die einer Folie, kann auch dadurch erfolgen, daß man auf diesen bzw. auf sie Preßkraft zur Einwirkung bringt.

Die Strukturumwandlung erfindungsgemäßer Formkörper durch Einwirkung von Wärme oder chemischem Medium, wie vorstehend angegeben, auf diese kann dadurch unterstützt werden, daß man diese Einwirkungsmaßnahmen jeweils mit Krafteinwirkung kombiniert.

Bei der Strukturumwandlung von Formkörpern durch Beaufschlagung derselben mit gasförmigen oder flüssigen Medien, die das Copolymerisat anzulösen vermögen, aus dem diese jeweils bestehen, sind als solche beispielsweise Tetrahydrofuran, niedermolekulare aliphatische Alkohole, inbesondere auch Aceton, geeignet. Die bezeichneten chemischen Medien werden bevorzugt in Dampfform auf die Folie zur Einwirkung gebracht.

Je nach Intensität und/oder Einwirkungsdauer der Strukturumwandlungsmaßnahme(n) auf die Formkörper wird deren Struktur je nach Bedarf mehr oder minder weitgehend und im Grenzfall bis zur physikalisch praktisch homogenen Stufe umgewandelt.

Bei Anwendung von Temperatur als Umwandlungsmaßnahme kann je nach deren Höhe und deren Dauer das Ausmaß der Strukturumwandlung gezielt eingestellt werden. Bei ausreichender Temperatureinwirkung erfolgt die Strukturumwandlung praktisch vollständig, bei kurzer nur teilweise.

Bei Verwendung gasförmiger oder flüssiger Medien zur Strukturumwandlung ist es möglich, durch deren Konzentration und Einwirkungsdauer die Strukturumwandlung zu steuern.

Die Strukturumwandlung erfindungsgemäßer Formkörper durch Einwirkung von Wärme auf diese, kann beispielsweise derart erfolgen, daß man sie mit heißer Luft hinreichender Temperatur beaufschlagt oder sie der Einwirkung von Ultrarotstrahlung unterwirft. Bei einem Formkörper, der als copolymerisierte Komponente Ethylen enthält, beispielsweise einer Folie, die eine Dicke von beispielsweise 30 $\mu$m hat, erfolgt die Strukturumwandlung dadurch, daß man sie einer Wärmeeinwirkung unterwirft, die sie auf eine Temperatur von ca. 90 °C erhitzt. Die Strukturumwandlung erfolgt dabei innerhalb eines Zeitraumes von ca. 5 s.

Die Strukturumwandlung zu einer optisch homogenen, d. h. transparenten Folie kann auch dadurch erfolgen, daß in die Poren erfindungsgemäßer Formkörper chemisch inerte flüssige Medien, beispielsweise Paraffine oder halogenierte Kohlenwasserstoffe oder auch Wasser eingelagert werden. Als Paraffin ist beispielsweise Dodecan, als halogenierter Kohlenwasserstoff beispielsweise Trichlorethan oder Methylenchlorid geeignet. Infolge ihres Dampfdruckes verflüchtigen sich die bezeichneten flüssigen Medien jeweils innerhalb bestimmter von ihrem Dampfdruck abhängigen Zeiträumen aus den Poren. Die Formkörper sind nach Beladung ihrer Poren mit den angegebenen flüssigen Medien innerhalb der Bereiche, in denen die Poren mit genannten Medien gefüllt sind, transparent. Nach Maßgabe der Verdampfungsdauer der Medien aus den mit diesen gefüllten Poren der Formkörper werden diese gezielt nach längerer oder kürzerer Zeitdauer wieder weiß. Diese reversible Strukturumwandlung kann (insbesondere in segmentierten Zonen) in Abhängigkeit vom eingelagerten Fluid auch durch Anlegen physiko-chemischer Gradienten (z. B. Temperatur, Licht, elektrisches Potential) beschleunigt oder gesteuert werden. Der genannte Effekt wird beispielsweise bei der Verwendung erfindungsgemäßer Folien in der Weise ausgenutzt, daß man in diesen transparente Zonen ausbildet, die sich optisch wahrnehmbar von ihrer weißen Umgebung unterscheiden, indem man die Folien segmentär mit bezeichneten inerten flüssigen Medien beaufschlagt, beispielsweise dadurch, daß man mit einem mit vorstehend genannter Flüssigkeit befeuchteten Pinsel beispielsweise Buchstaben, Zahlen oder Ornamente aufbringt oder sichtbar werden läßt. Bei Wahl geeigneter flüssiger Medien verdampfen diese je nach ihrem Dampfdruck mehr oder weniger schnell aus den Poren der Folie, so daß diejenigen Zonen, die zuvor infolge von Flüssigkeit in den Poren transparent waren, nach mehr oder minder kurzer Zeit wieder weiß werden.

Wenn man als flüssiges Schreibmittel eine Flüssigkeit, die befähigt ist, eine bleibende Strukturumwandlung der Folie zu bewirken, auf die weiße Folie segmentär aufbringt, wird der flüssigkeitsbeaufschlagte Bereich irreversibel transparent, d. h. in dieser Weise auf die Folie aufgebrachte Informationen bleiben dauerhaft erhalten.

Bei der erstgenannten Anwendung ist die Folie z. B. als Schreibfolie oder Informationsträger bzw. -vermittler mehrfach zu verwenden, bei der zweiten nur einmal.

Zur Herstellung erfindungsgemäßer Formkörper geht man von Lösungen aus, die als erfindungsessentielle Bestandteile Copolymerisate des vorstehend angegebenen qualitativen und quantitativen chemischen Aufbaus enthalten.

Nachfolgend wird beispielhaft die Herstellung erfindungsgemäßer Formkörper angegeben :

Man geht von einer flüssigen Lösung aus, die 1 bis 50 Gew. %, bezogen auf das Gesamtgewicht der Lösung, Polymerisat als gelösten Anteil enthält, wobei der gelöste Anteil zu wenigstens 70 Gew. %, bezogen auf die Gesamtmenge des in der Flüssigkeit gelösten Polymerisats, aus Copolymerisat besteht, das zu 20 bis 80 Gew. %, bevorzugt zu 30 bis 70 Gew. %, aus copolymerisiertem fluoriertem Olefin, zu 0 bis 40 Gew. % aus copolymerisiertem Olefin und zu 80 bis 20 Gew. %, bevorzugt zu 70 bis 30 Gew. %, aus copolymerisiertem Vinylacetat aufgebaut ist, wobei wenigstens 5 %, vorteilhaft über 80 %, der Acetatgruppen des angegebenen Copolymerisats, bezogen auf deren Anteil in diesem, nach erfolgter Copolymerisation der genannten Comonomeren zum bezeichneten Copolymerisat, durch Verseifung in OH-Gruppen umgewandelt sind.

Gegebenenfalls kann die Lösung bis zu 30 Gew. %, bezogen auf das Gesamtgewicht der in ihr gelösten Polymerisate, beispielsweise Polyvinylidenfluorid, polares Polyolefin oder Silikone oder Abmischungen dieser Polymerisate enthalten, die sich in ihrem qualitativen chemischen Aufbau von dem der bezeichneten Copolymerisate unterscheiden.

Bevorzugt enthält die zur Herstellung der Formkörper verwendete Lösung 5 bis 25 Gew. %, bezogen auf ihr Gesamtgewicht, gelösten Anteil.

Geeignete Lösungsmittel zur Herstellung der Lösung sind bspw. Dimethylformamid, N-Methylpyrrolidon, Dimethylsulfoxid, Dimethylacetamid, aliphatische Alkohole, Aceton oder Tetrahydrofuran.

Die bezeichnete Lösung wird in Form eines flüssigen Filmes aus dem geraden Schlitzspalt eines Düsenkörpers oder in Form eines flüssigen Hohlfadens aus dem Ringschlitzspalt eines Düsenkörpers jeweils aus- und in Fällflüssigkeit eingepreßt ; vor Eintritt in das Fällbad kann z. B. auch eine Verweildauer an der Luft vorgeschaltet werden. In der Fällflüssigkeit ist das in der Lösung gelöste Copolymerisat unlöslich, das Lösungsmittel jedoch löslich. Als Fällflüssigkeit wird beispielsweise Wasser verwendet.

Die Fällflüssigkeit koaguliert bei ihrer Einwirkung (in Abhängigkeit z. B. von der Fällbadtemperatur) auf den flüssigen Film bzw. auf den flüssigen Hohlfaden, das in diesem enthaltene Copolymerisat unter Ausbildung einer formbeständigen Folie bzw. unter Ausbildung eines formbeständigen Hohlfadens aus bezeichnetem Copolymerisat, die bzw. der die angegebene Struktur besitzt.

Der Film bzw. der Hohlfaden wird dann durch Trocknung von überschüssiger Flüssigkeit befreit oder die Fällflüssigkeit durch eine andere Flüssigkeit (z. B. Glycerin) ersetzt.

Eine Folie gemäß der Erfindung kann auch in der Weise hergestellt werden, daß man eine flüssige Schicht aus vorstehend angegebener Copolymerisatlösung auf die Oberfläche einer formstabilen Trägerfolie, beispielsweise in Gestalt eines Metallbandes, aufbringt und dann Fällflüssigkeit auf die auf der Trägerfolie befindliche flüssige Schicht zur Einwirkung bringt und den dabei gebildeten Film aus angegebenem Copolymerisat von der Trägerfolie abzieht.

Wenn als Trägerfolie beispielsweise eine streckorientierte Polyesterfolie oder eine Folie aus Hart-PVC verwendet wird, wobei sie beispielsweise eine Dicke im Bereich von 50 bis 100 µm besitzt und man im übrigen wie vorstehend angegeben verfährt, kann man nach Ausbildung des Copolymerisatfilmes auf der Trägerfolienoberfläche nach Trocknung des Filmes das zweilagige Laminat erfindungsgemäß verwenden.

Die Poren erfindungsgemäßer Formkörper sind mit Fluid beladbar, d. h. mit diesem ausfüllbar.

Unter Fluiden sind definitionsgemäß Flüssigkeiten zu verstehen, gegen die das Copolymerisat, das die erfindungsgemäße Folie bildet, beständig ist, so daß es von diesen praktisch nicht angelöst wird.

Fluide können chemisch einheitliche Flüssigkeiten oder Lösungen bzw. Gemische aus solchen sein.

Fluide können auch aus Flüssigkeiten bestehen, die chemische Stoffe gelöst enthalten. Das Lösungsmittel ist dabei der Träger für den in ihm gelösten chemischen Wirkstoff, der mit diesem und durch dieses in die Poren der Folie gelangt, wenn man die Folie mit dem Fluid beaufschlagt und dadurch die Poren mit diesem ausfüllt. Fluide können auch aus Flüssigkeiten bestehen, die chemische Wirkstoffe in dispergierter oder emulgierter Form enthalten ; auch in diesem Fall ist die kontinuierliche Phase des Fluids der Träger für das in ihr Emulgierte oder Dispergierte. Der flüssige Träger bewirkt, daß die in ihm enthaltenen Wirkstoffe in die Poren der erfindungsgemäßen Folie gelangen, wenn man die Folie mit angegebenen Fluiden beaufschlagt.

Ein Beispiel für ein Fluid der erstgenannten Art ist eine organische flüssig-kristalline Phase von 4-Methoxybenzyliden-4'-n-butylanilin (Smp. 21 °C).

Ein Beispiel für Fluide der zweiten Gattung ist ein in organischer Phase (z. B. Dodecan) gelöstes photosensitives Sulfonamid des o-Naphtochinondiazides.

Ein Beispiel für ein Fluid mit dispergiertem bzw. emulgiertem Wirkstoffanteil ist eine wäßrige Suspension des Schilddrüsenhormones L-Thyroxin.

Fluide können auch zwei oder mehrere chemisch unterschiedliche Stoffe als Wirkstoff gelöst, dispergiert oder emulgiert enthalten.

Definitionsgemäß soll die Bezeichnung « Fluide » im weiteren Sinne auch Pasten sowie Gele umfassen, wobei deren flüssiger Anteil das Fluid im engeren Sinne ist, da nur dieser in die Poren der erfindungsgemäßen Folie eindringt, wenn man diese mit der Paste oder dem Gel beaufschlagt oder in Kontakt bringt.

Geeignete Gele sind beispielsweise solche auf Basis von Agarose. Sie können beispielsweise einen Wasseranteil von ca. 300 % oder größer, bezogen auf das Gewicht des gelbildenden Polymeren, enthalten ; der wäßrige Anteil des Gels enthält chemischen Wirkstoff, beispielsweise Scopolamin oder Nitroglycerinderivat gelöst.

Zur Beladung der Poren mit Fluid kann die Folie beispielsweise in eine mit Fluid gefüllte Wanne eingetaucht werden. Nach Entnahme der Folie aus der Wanne kann sie von auf ihrer Oberfläche befindlichem überschüssigem Fluid befreit werden, beispielsweise durch Abstreifen, Abpressen oder Abwischen desselben.

Man kann die Poren der Formkörper auch nur innerhalb segmentärer Bereiche derselben mit Fluid beladen. Dies soll am Beispiel einer erfindungsgemäßen Folie erläutert werden. Man geht dazu in folgender Weise vor : Man füllt zunächst sämtliche Poren der Folie in vorstehend angegebener Weise mit Fluid. Danach wandelt man die Struktur der Folie innerhalb eines segmentären Bereiches derselben um, indem man beispielsweise Acetondampf auf diesen einwirken läßt. Innerhalb des strukturumgewandelten segmentären Folienbereiches ist Fluid in der Folie immobilisiert enthalten.

Zweckmäßig geht man dabei derart vor, daß man die insgesamt mit Fluid beladene Folie vor der Strukturumwandlungsmaßnahme mit einer Maske abdeckt, die in Form und Abmessung der Folie entspricht. Die Maske hat verschieden gestaltete Ausnehmungen, beispielsweise ein Raster aus rechteckigen Öffnungen. Man läßt dann Acetondampf auf die Maske in der Weise einwirken, daß der von ihr nicht abgedeckte Folienbereich mit diesem beaufschlagt wird.

Die Strukturumwandlung des beaufschlagten segmentären Folienbereiches erfolgt bei Verwendung von gesättigtem Acetondampf innerhalb von 30 bis 60 s.

Man entfernt dann die Maske von der Folie und eluiert dann mit einem geeigneten flüssigen Elutionsmittel das Fluid aus den offenen Poren der Folie. Das in den segmentären strukturumgewandelten Bereichen der Folie enthaltende Fluid wird dabei aus diesen nicht eluiert.

Die Folie wird sodann zur erneuten Befüllung ihrer verbliebenen Poren mit Fluid in angegebener Weise behandelt. Das im zweiten Verfahrensschritt verwendete Fluid unterscheidet sich chemisch von dem im ersten Verfahrensschritt verwendeten.

Danach wandelt man die Struktur der Folie in vorstehend angegebener Weise um.

Das Verfahrensprodukt enthält dann zwei Fluide, die sich chemisch voneinander unterscheiden, jeweils in definierten Bereichen der Folie, beispielsweise zur Inkorporation zweier an sich unverträglicher Substanzen in eine Folie.

Man kann das Verfahren auch derart durchführen, daß man nach der ersten Beladung der Folie mit Fluid innerhalb segmentärer Bereiche derselben, die Strukturumwandlung derart führt, daß die gesamte Folie strukturumgewandelt wird. Das Verfahrenserzeugnis ist dann eine transparente Folie, die innerhalb eines diskreten Bereiches Fluid immobilisiert enthält.

Wenn man als Fluid beispielsweise flüssige, flüchtige Aromastoffe oder Lösungen, die Aromastoffe gelöst enthalten, verwendet, oder flüssige, flüchtige Insektizide oder Lösungen, die diese enthalten, einsetzt, sind diese eingeschlossen ; die bezeichneten chemischen Wirkstoffe treten infolge Diffusion langsam aus der physikalisch praktisch homogenen Folie bzw. den segmentären Bereichen derselben aus und entwickeln außerhalb derselben die erwünschte Wirkung, die entsprechend der Diffusionsgeschwindigkeit der Stoffe langzeitig ist. Sofern man als Fluid ein solches verwendet, das licht- oder wärmeempfindliche chemische Verbindungen enthält oder reaktive Verbindungen, die unter Einwirkung eines elektrischen Potentials ihre Struktur bzw. Farbe verändern, kann eine erfindungsgemäße Folie, die derartige Fluide immobilisiert enthält, auf dem Gebiet der Reproduktionstechnik Anwendung finden oder für phototechnische Verfahren eingesetzt werden.

Die Farbe bzw. die Farbintensität von Folienbereichen, die durch Strukturumwandlung in angegebener Weise physikalisch praktisch homogen sowie transparent erscheinen und gegebenenfalls Fluid immobilisiert enthalten, entspricht der Farbe bzw. der Farbintensität der in ihnen enthaltenen Fluide bzw. den farbgebenden chemischen Stoffen in diesen.

Nachfolgend werden besondere Verwendungen erfindungsgemäßer Folien beispielhaft angegeben :

1. Verwendung der erfindungsgemäßen Folie als temporäres Depot und Umhüllung für Drogen, Katalysatoren, Enzyme, Insektizide, Farbstoffe, Flüssigkristalle, Korrosionsinhibitoren oder zur Abdeckung sterilisierter Güter.

Beispiel

Eine wäßrige gepufferte Suspension mit 50 mg Pilocarpin wird in einen Beutel aus 5 cm² erfindungsgemäßer Folie, die aus Copolymerisat (3) besteht, eingefüllt und dicht verschlossen. Der Beutel wird dann 30 s einer gesättigten Atmosphäre aus Acetondampf ausgesetzt. Die ursprünglich weiße Folie wird dabei opak-durchscheinend. Dieser Vorgang geht mit einer definierten Verengung der Poren und damit gewünschten Einstellung ihrer Permeabilität einher. Der Beutel wird sodann in 100 ml gerührte Pufferlösung eingebracht und in dieser aufgehängt. Die Kinetik der Drogenabgabe aus dem Beutel in die ihn umgebende Flüssigkeit wird gemessen. Nach einer Anlaufphase stellt sich bei einer Abgabegeschwindigkeit von 16 mg Wirkstoff pro Woche die erforderliche konstante Kinetik 0. Ordnung ein.

2. Verwendung der Folie für analytische und diagnostische Verfahren, wie Immundiffusion, Immunoelektrophorese, Radioimmunoassay, Agglutinationstests sowie Diagnosesticks.

Beispiel

5 ml einer Antigenlösung aus wäßrigem gepuffertem Humanalbumin (8 μg) werden in ein Probeapplikationsloch in eine Copolymerisatfolie nach (5) eingebracht. Die Folie ist in einer Schichtstärke von 400 μm auf einer Polyesterfolie (Hostaphan® 100 μm) aufgetragen und wurde zuvor mit 14 % Antihumanalbuminserum (Kaninchen) getränkt. Nach einer Diffusionszeit von 24 Stunden in einer feuchten Kammer wird die Schicht in physiologischer NaCl-Lösung für 48 Stunden eluiert und direkt anschließend mit einem Proteinfarbstoff (0,1 % Coomassieblau) angefärbt und in Acetondampf für 60 s fixiert.

Es liegt eine transparente Folie vor, die eine deutlich ausgeprägte radiale Präzipitatzone aufweist und sich in Transmission quantitativ auswerten läßt.

3. Verwendung als reprographischer und optischer Informationsträger, beispielsweise für Fotokopien mit Naß- und Trockentonern (insbesondere für die Overheadprojektion), als Zeichenfolie, als Substrat für reprographische Folien (insbesondere als Träger photoaktiver Substanzen), als Thermopapier (beispielsweise für Thermokopierer), als « Blende » zum Abrufen von Texten und optischer Information, zum zeitlich begrenzten Abschirmen lichtempfindlicher Schichten (beispielsweise von Film und Photomaterial), bzw. umgekehrt zum zeitlich begrenzten Belichten von elektrooptischen oder reaktiven Systemen, zum zeitlich definierten Sichtbarmachen von Informationen, Gegenständen, Räumen oder als Indikator von Dämpfen oder Fluiden sowie spezifischer Temperaturen.

Beispiel a)

Eine weiße, 50 μm starke Schicht einer Terpolymerisatfolie nach (4 bzw. 3) auf Polyesterfolie (Hostaphan® 100 μm) wird auf einem Overheadprojektor mit einem Filzstift beschriftet, der mit Dodecan getränkt ist. Es entsteht ein transparentes bzw. die Farbe des Untergrundes wiedergebendes, scharf umrissenes Schriftbild in weißer Umgebung, das nach Übermittlung der gewünschten Information nach wenigen Minuten verschwindet, so daß die Folie erneut einsetzbar ist.

Beispiel b)

Eine weiße, 30 μm starke Schicht aus Terpolymerisatfolie nach (5) wird auf einer Photokopiermaschine (Infotec 1801) nach einer technischen Testvorlage naß getont und anschließend thermisch bei etwa 90 °C fixiert. Es entsteht eine kratzfeste transparente Folie, die sich durch sehr hohes Auflösungsvermögen der Testlinien auszeichnet und als Vorlage für die Overheadprojektion geeignet ist.

Da die erfindungsgemäßen Folien bzw. rohrartigen Gebilde schweißbar sind, lassen sich aus ihnen in einfacher Weise Folien- bzw. Schlauchbeutel herstellen ; dazu geht man beispielsweise in der Weise vor, daß man zwei Folienstücke jeweils gleicher Form und Abmessung derart übereinanderlegt, daß ihre Ränder fluchten. Die Folienstücke werden dann im Bereich ihrer Ränder miteinander verschweißt. Dabei wird zunächst ein an einer Seite offener Beutel gebildet, dieser wird mit Füllgut befüllt und sodann in angegebener Weise verschlossen. Bei Verwendung eines Schlauchstückes wird dieses zunächst an seinem einen Ende verschlossen, sein Hohlraum wird dann mit Füllgut befüllt, danach wird das Schlauchstück auch am anderen Ende verschlossen ; das Verschließen erfolgt jeweils durch Verschweißung.

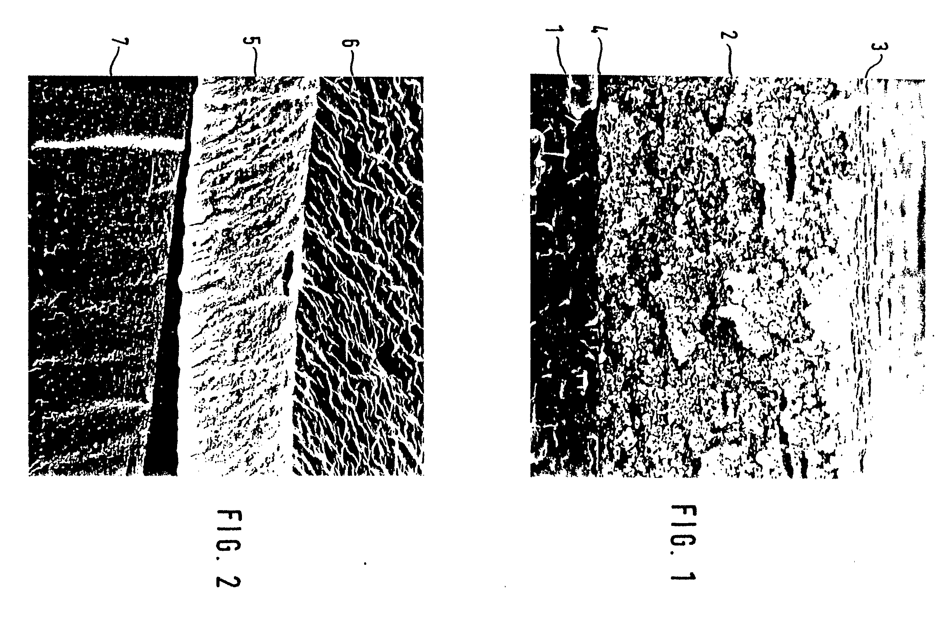

Die gegenständliche Erfindung wird durch die Figuren 1 und 2 jeweils beispielhaft erläutert.

Figur 1 zeigt eine Photographie einer Folie gemäß der Erfindung im Querschnitt in 300-facher Vergrößerung.

Fig. 2 zeigt eine Photographie einer Folie mit physikalisch praktisch homogener Struktur, die durch Strukturumwandlung einer in Fig. 1 dargestellten Folie hergestellt ist (300-fache Vergrößerung).

In Fig. 1 bedeuten
1 eine Trägerfolie,
2 die poröse Folie,
3 die freiliegende Oberfläche der Folie 2 und
4 die Grenzfläche zwischen poröser Folie 2 und Trägerfolie 1.
In Fig. 2 bedeutet
5 eine Folie mit physikalisch praktisch homogener Struktur,

6 ist ihre Oberfläche ; die Folie 5 ist im von der Kunststoff-Trägerfolie 7 praktisch abgelösten Zustand dargestellt.

Die Bezeichnung « flüssiger Film » bzw. « flüssiges rohrförmiges Gebilde » soll im Rahmen der Erfindungsbeschreibung und den Ansprüchen jeweils die Bedeutung « Film aus Flüssigkeit » bzw. « rohrförmiges Gebilde aus Flüssigkeit » haben.

**Patentansprüche**

1. Formkörper offenporiger Struktur aus thermoplastischem Kunststoff, dadurch gekennzeichnet, daß er inhärente latente Strukturumwandelbarkeit sowie effektive Poren eines Durchmessers im Bereich von 0,002 bis 10 μm besitzt und zu wenigstens 70 Gew.-% aus Copolymerisat besteht, das zu 20 bis 80 Gew.-%, bezogen auf das Gesamtgewicht des Copolymerisates, aus copolymerisiertem fluoriertem Olefin, zu 0 bis 40 Gew.-%, bezogen auf sein Gesamtgewicht, aus copolymerisiertem Olefin und zu 80 bis 20 Gew.-%, bezogen auf das Gesamtgewicht des Copolymerisates, aus copolymerisiertem Vinylacetat aufgebaut ist, wobei wenigstens 5 %, vorteilhaft über 80 %, der Acetatgruppen des angegebenen Copolymerisates, bezogen auf ihren Gesamtanteil in diesem, nach erfolgter Copolymerisation der genannten Comonomeren zum bezeichneten Copolymerisat durch Verseifung in OH-Gruppen umgewandelt sind.

2. Formkörper nach Anspruch 1, dadurch gekennzeichnet, daß das ihn bildende Copolymerisat zu 30 bis 70 Gew.-%, bezogen auf dessen Gesamtgewicht aus copolymerisiertem Tetrafluorethylen, zu 0 bis 20 Gew.-% aus copolymerisiertem Ethylen und zu 70 bis 30 Gew.-%, bezogen auf das Gesamtgewicht des Copolymerisates, aus copolymerisiertem Vinylacetat besteht, wobei wenigstens 5 % der Acetatgruppen des Copolymerisates vorteilhaft mehr als 80 % derselben, nach erfolgter Copolymerisation der angegebenen Comonomeren zu bezeichnetem Copolymerisat durch Verseifung in OH-Gruppen umgewandelt sind.

3. Formkörper nach Anspruch 1 und 2 in Form einer Folie.

4. Formkörper nach Anspruch 1 und 2 in Form eines rohrförmigen Gebildes.

5. Verfahren zur Herstellung von Formkörpern nach Anspruch 1 bis 4, dadurch gekennzeichnet, daß man aus flüssiger, 1- bis 50-gewichtsprozentiger Lösung, bezogen auf das Gesamtgewicht der Lösung, die als gelösten Anteil Polymeres enthält, das zu wenigstens 70 Gew.% aus Copolymerisat besteht, das zu 20 bis 80 Gew.-%, bezogen auf das Gesamtgewicht des Copolymerisates aus copolymerisiertem fluoriertem Olefin, zu 0 bis 40 Gew.-%, bezogen auf sein Gesamtgewicht, aus copolymerisiertem Olefin und 80 bis 20 Gew.-%, bezogen auf das Gesamtgewicht des Copolymerisates aus copolymerisiertem Vinylacetat aufgebaut ist, wobei wenigstens 5 %, vorteilhaft über 80 % der Acetatgruppen des angegebenen Copolymerisates, bezogen auf ihren Anteil an diesem, nach erfolgter Copolymerisation der genannten Comonomeren zum bezeichneten Copolymerisat durch Verseifung in OH-Gruppen umgewandelt sind, einen flüssigen Film bzw. ein flüssiges rohrförmiges Gebilde formt, auf diesen bzw. dieses jeweils Fällflüssigkeit zur Einwirkung bringt, durch deren Wirkung das im flüssigen Film bzw. im rohrförmigen Gebilde gelöste Copolymerisat zu einer porös-strukturierten formstabilen Folie bzw. einem porös-strukturierten formstabilen rohrförmigen Gebilde koaguliert wird.

6. Verfahren nach Anspruch 5, dadurch gekennzeichnet, daß man als Polymeren-Lösung eine solche einsetzt, die als gelösten Anteil Copolymerisat enthält, das zu 30 bis 70 Gew. %, bezogen auf dessen Gesamtgewicht, aus copolymerisiertem Tetrafluorethylen, zu 0 bis 20 Gew. %, bezogen auf sein Gesamtgewicht, aus copolymerisiertem Ethylen und zu 70 bis 30 Gew. %, bezogen auf sein Gesamtgewicht aus copolymerisiertem Vinylacetat besteht, wobei wenigstens 5 % der Acetatgruppen des Copolymerisats, vorteilhaft mehr als 80 % derselben, nach erfolgter Copolymerisation der angegebenen Comonomeren zu bezeichnetem Copolymerisat, durch Verseifung in OH-Gruppen umgewandelt sind.

7. Verwendung einer Folie nach Anspruch 1 und 2 als Depot und zur Umhüllung für chemische Mittel.

8. Verwendung einer Folie nach Anspruch 1 und 2 für analytische und diagnostische Verfahren.

9. Verwendung einer Folie als reprographischer oder optischer Informationsträger bzw. Informationsvermittler.

10. Verfahren zur Umwandlung der Struktur von Formkörpern eines Aufbaues entsprechend Anspruch 1 bis 4, bei dem man diese der Einwirkung von Wärme oder der Einwirkung von chemischen Medien in flüssiger Form oder in Form eines Gases unterwirft, die befähigt sind, die Struktur des die Formkörper jeweils bildenden Copolymerisates physikalisch bzw. optisch homogen zu machen oder auf sie Druck zur Einwirkung bringt oder sie zugleicher Einwirkung von Wärme und Druck oder zugleicher Einwirkung von bezeichneten chemischen Medien und Druck oder Wärme unterwirft.

11. Verfahren nach Anspruch 10, dadurch gekennzeichnet, daß man die chemischen Medien jeweils in Dampfform auf die Formkörper zur Einwirkung bringt.

**Claims**

1. Molded article having an open-cell structure and being made of a thermoplastic material, characterized in that it possesses an inherent latent structural convertibility and has effective pores of a diameter in the range from 0.002 to 10 μm and comprises at least 70 percent by weight of a copolymer which is composed of 20 to 80 percent by weight, relative to the total weight of the copolymer, of copolymerized fluorinated olefin, 0 to 40 percent by weight, relative to its total weight, of copolymerized olefin, and 80 to 20 percent by weight, relative to the total weight of the copolymer, of copolymerized vinyl acetate, with at least 5 percent, advantageously more than 80 percent, of the total proportion of acetate groups contained in the copolymer being converted by saponification into OH groups, after copolymerization of the specified comonomers to form the copolymer.

2. A molded article according to claim 1, characterized in that the copolymer of which it is formed is composed of 30 to 70 percent by weight, relative to its total weight, of copolymerized tetrafluoroethylene, 0 to 20 percent by weight of copolymerized ethylene, and 70 to 30 percent by weight, relative to the total weight of the copolymer, of copolymerized vinyl acetate, with at least 5 percent, advantageously more than 80 percent, of the acetate groups contained in the copolymer being converted by saponification into OH groups, after copolymerization of the specified comonomers to form the copolymer.

3. A molded article according to claims 1 and 2, which has the form of a film.

4. A molded article according to claims 1 and 2, which has the form of a tubular body.

5. Process for the preparation of molded articles according to claims 1 to 4, characterized in that a liquid solution of 1 to 50 weight percent strength, relative to its total weight, is employed, which contains as the dissolved constituent a polymer comprising at least 70 percent by weight of a copolymer composed of 20 to 80 percent by weight, relative to the total weight of the copolymer, of copolymerized fluorinated olefin, 0 to 40 percent by weight, relative to its total weight, of copolymerized olefin, and 80 to 20 percent by weight, relative to the total weight of the copolymer, of copolymerized vinyl acetate, with at least 5 percent, advantageously more than 80 percent, of the proportion of acetate groups contained in the copolymer being converted by saponification into OH groups, after copolymerization of the specified comonomers to form the copolymer, the solution being used to produce a liquid film or a liquid tubular body and precipitating liquid being allowed to act on the liquid film or liquid tubular body, by the action of which the copolymer dissolved in the liquid film or liquid tubular body is coagulated into a porous-structured, dimensionally stable film or into a porous-structured, dimensionally stable tubular body.

6. A process according to claim 5, in which the polymer solution used comprises as the dissolved constituent a copolymer which is composed of 30 to 70 percent by weight, relative to its total weight, of copolymerized tetrafluoroethylene, 0 to 20 percent by weight, relative to its total weight, of copolymerized ethylene, and 70 to 30 percent by weight, relative to its total weight, of copolymerized vinyl acetate, with at least 5 percent, advantageously more than 80 percent, of the acetate groups contained in the copolymer being converted by saponification into OH groups, after copolymerization of the indicated comonomers to form the copolymer.

7. Use of a film according to claims 1 and 2 as a deposit and an envelope for chemical media.

8. Use of a film according to claims 1 and 2 for analytical and diagnostic processes.

9. Use of a film as a reprographic or optical information carrier or information transmitting material.

10. Process for converting the structure of molded articles according to claims 1 to 4, in which the molded articles are subjected to the action of heat or to the action of chemical media in a liquid or gaseous form, which are capable of rendering the structure of the copolymer which, in each case, forms the molded articles, physically and/or optically homogeneous, or pressure is allowed to act on the molded articles, or the molded articles are subjected to a simultaneous action of heat and pressure or to a simultaneous action of specified chemical media and pressure or heat.

11. A process according to claim 10, in which the chemical media which are allowed to act on the molded articles are in a vaporous form.

**Revendications**

1. Article moulé ayant une structure à cellules ouvertes en matière thermo-plastique, caractérisé en ce qu'il possède une convertibilité structurale latente inhérente ainsi que des pores utiles d'un diamètre dans la gamme de 0,002 à 10 μm et est constitué d'au moins 70 % en poids d'un copolymère qui est composé de 20 à 80 % en poids, par rapport au poids total du copolymère, d'une oléfine fluorée copolymérisée, de 0 à 40 % en poids, par rapport à son poids total d'une oléfine copolymérisée et de 80 à 20 % en poids, par rapport au poids total du copolymère, d'acétate de vinyle copolymérisé, au moins 5 %, et de façon avantageuse plus de 80 %, de la proportion totale des groupes acétates contenus dans le copolymère étant transformés par saponification en groupes OH, après la copolymérisation des comonomères indiqués pour former le copolymère.

2. Article moulé selon la revendication 1 caractérisé en ce que le copolymère dont il est formé est composé de 30 à 70 % en poids, par rapport à son poids total, de tétrafluoroéthylène copolymérisé, de 0 à 20 % en poids d'éthylène copolymérisé et de 70 à 30 % en poids, par rapport au poids total du copolymère, d'acétate de vinyle copolymérisé, au moins 5 %, et de façon avantageuse plus de 80 % des groupes acétate contenus dans le copolymère étant transformés par saponification en groupes OH, après copolymérisation des comonomères indiqués pour former le copolymère.

3. Article moulé selon les revendications 1 et 2 sous la forme d'une pellicule.

4. Article moulé selon les revendications 1 et 2 sous la forme d'un élément tubulaire.

5. Procédé pour la préparation d'articles moulés selon les revendications 1 à 4, caractérisé en ce qu'à partir d'une solution liquide ayant une concentration de 1 à 50 % en poids, par rapport à son poids total, qui contient comme constituant dissous un polymère constitué d'au moins 70 % en poids d'un copolymère composé de 20 à 80 % en poids, par rapport au poids total du copolymère, d'une oléfine fluorée copolymérisée, de 0 à 40 % en poids, par rapport à son poids total, d'une oléfine copolymérisée et de 80 à 20 % en poids, par rapport au poids total du copolymère, d'acétate de vinyle copolymérisé, au moins 5 %, et de façon avantageuse plus de 80 %, de la proportion des groupes acétate contenus dans le copolymère étant transformés par saponification en groupes OH après copolymérisation des comonomères indiqués pour former le copolymère, on forme une pellicule liquide ou un élément tubulaire liquide et on fait agir, sur la pellicule liquide ou l'élément tubulaire liquide, un liquide précipitant, sous l'action duquel le copolymère dissous dans la pellicule liquide ou l'élément tubulaire liquide est coagulé en une pellicule de structure poreuse de dimensions stables ou en un élément tubulaire de structure poreuse de dimensions stables.

6. Procédé selon la revendication 5 caractérisé en ce qu'on utilise, comme solution de polymère, une solution qui comprend, comme constituant dissous, un copolymère qui est composé de 30 à 70 % en poids, par rapport à son poids total, de tétrafluoroéthylène copolymérisé, de 0 à 20 % en poids, par rapport à son poids total, d'éthylène copolymérisé et de 70 à 30 % en poids, par rapport à son poids total, d'acétate de vinyle copolymérisé, au moins 5 %, et de façon avantageuse plus de 80 % des groupes acétate contenus dans le copolymère étant transformés par saponification en groupes OH, après copolymérisation des comonomères indiqués pour former le copolymère.

7. Utilisation d'une pellicule selon les revendications 1 et 2 comme dépôt et pour l'emballage de milieux chimiques.

8. Utilisation d'une pellicule selon les revendications 1 et 2 pour des procédés d'analyse et de diagnostic.

9. Utilisation d'une pellicule comme support d'information reprographique ou optique ou matériau de transmission d'informations.

10. Procédé pour la transformation de la structure d'articles moulés selon les revendications 1 à 4 dans lequel on soumet les articles moulés à l'action de la chaleur ou à l'action de milieux chimiques, sous une forme liquide ou gazeuse, capables de rendre la structure du copolymère, qui dans chaque cas forme les articles moulés, physiquement et/ou optiquement homogène, ou bien on laisse une pression s'exercer sur les articles moulés, ou bien on soumet les articles moulés à une action simultanée de la chaleur et de la pression ou à une action simultanée de milieux chimiques déterminés et de pression ou de chaleur.

11. Procédé selon la revendication 10 caractérisé en ce qu'on fait agir les milieux chimiques à l'état de vapeur sur les articles moulés.

FIG. 1

FIG. 2